# EUROPEAN PATENT APPLICATION

(11) **EP 1 062 953 A1**
(43) Date of publication of application: **27.12.2000**
(21) Application number: 99202004.0
(22) Date of filing: 22.06.1999
(51) Int. Cl.: A61K 39/395, A61K 38/48

(54) **Use of compounds that reduce alpha2-antiplasmin in vivo for the preparation of a composition for the treatment of ischemic stroke**

(71) Applicant: Leuven Research & Development vzw, 3000 Leuven (BE)
(72) Inventor: Nagai, Nobuo, Shizuoka 431-3124 (JP); Collen, Desiré José, London SW5 0HN (GB)
(74) Representative: Van Someren, Petronella F. H. M.

(57) **Abstract**

The present invention relates to a new means for the treatment of focal ischemic cerebral infarction (ischemic stroke). It has been found that reduction of α₂-antiplasmin leads to a significantly smaller focal cerebral infarct size. The invention therefore provides the use of compounds that reduce α₂-antiplasmin concentration or activity in vivo, for the preparation of a therapeutical composition for the treatment of focal cerebral ischemic infarction (ischemic stroke).

## Description

The present invention relates to a new means for the treatment of focal ischemic cerebral infarction (ischemic stroke).

Focal ischemic cerebral infarction occurs when the arterial blood flow to a specific region of the brain is reduced below a critical level resulting in neuronal cell death. It is thought that neuronal degeneration in central nervous system (CNS) diseases such as stroke, epilepsy and Alzheimer's disease is stimulated by an excess of the excitatory amino acid glutamate (2). Injection of glutamate agonists in the CNS indeed induces hippocampal neuronal cell death similar to that observed in neurodegenerative diseases (3).

Excitotoxin-induced neuronal degeneration is mediated by tissue-type plasminogen activator (t-PA) (4). Consistent with this observation, mice deficient in t-PA are resistant to, and infusion of plasminogen activator inhibitor-1 (PAI-1) protects against excitotoxin-mediated hippocampal neuronal degeneration (4-6).

Furthermore, deficiency of plasminogen (Plg), the zymogen substrate of t-PA, and infusion of α₂-antiplasmin (α₂-AP), protect mice against excitotoxin-induced hippocampal neuronal death (5). It has been proposed that plasmin-mediated degradation of laminin sensitizes hippocampal neurons to cell death by disrupting neuron-extracellular matrix interaction (7).

Wang et al. (8) recently demonstrated that neuronal damage after focal cerebral ischemia induced by transient occlusion of the middle cerebral artery was also reduced in mice with t-PA deficiency and exacerbated by t-PA infusion. This suggests that the plasminogen system may be involved both in establishing a cerebral ischemic infarct and in its extension during thrombolytic therapy. It was recently demonstrated that the neurotoxic effect of t-PA on persistent focal cerebral ischemia also occurred with other thrombolytic agents, including streptokinase and staphylokinase (9). Thus, in those patients with persistent cerebral arterial occlusion, thrombolytic therapy for ischemic stroke may cause infarct extension, which would not only partially offset the established overall beneficial effect of arterial recanalization (10, 11), but indeed be harmful to a subgroup of patients. Because it is not possible to distinguish between patients who will and those who will not achieve cerebral arterial recanalization with thrombolytic therapy, the development of specific conjunctive strategies to counteract the neurotoxic effects of thrombolytic agents on persisting focal cerebral ischemia appear to be warranted.

It is therefore the object of the present invention to provide a new means for treating ischemic stroke.

In the research that led to the present invention the following was contemplated. Although it is assumed that neuronal injury during focal ischemia in the brain occurs primarily as a result of accumulation of excitotoxins such as glutamates, the role of plasmin-mediated laminin degradation or alternative mechanisms in the pathogenesis of cortical neuronal cell death has not been demonstrated. In order to delineate the contribution of individual components of the plasminogen (fibrinolytic) system on focal cerebral ischemic infarction, the present inventors then quantitated infarct size produced by ligation of the left middle cerebral artery (MCA) in mice with targeted inactivation of the genes encoding Plg, its activators tissue-type plasminogen activator (t-PA) or urokinase-type plasminogen activator (u-PA), or the fibrinolytic inhibitors PAI-1 or α₂-AP. In addition, the effects of adenoviral transfer of the t-PA and PAI-1 genes and of infusion of human α₂-AP on cerebral infarction were studied.

Whereas the findings of Strickland et al., that t-PA deficiency protects against focal cerebral ischemic infarction were fully confirmed, and extended by the observation that PAI-1 deficiency resulted in significantly larger infarct sizes, the observation that Plg deficiency protects against excitotoxin induced neuronal cell death could not be confirmed. Instead it was found that focal cerebral infarct size was significantly larger in mice with Plg deficiency and conversely, significantly smaller in mice with α₂-AP deficiency.

In aggregate, these findings indicate that plasminogen system components affect focal cerebral ischemic infarct size at two different levels: 1) reduction of t-PA activity (t-PA gene inactivation or PAI-1 gene transfer) reduces, while its augmentation (t-PA gene transfer or PAI-1 gene inactivation) increases infarct size, and 2) reduction of Plg activity (Plg gene inactivation or α₂-AP injection) increases, while its augmentation (α₂-AP gene inactivation or α₂-AP neutralization) reduces infarct size. The findings are incompatible with a unique linked pathway in which t-PA-mediated plasmin generation would lead to neuronal cell death, but suggests two independent (t-PA mediated and Plg-mediated, respectively) mechanisms operating in opposite direction.

The internally consistent observations with α₂-AP were unexpected but are most relevant for the treatment of ischemic stroke. Firstly a correlation was found between infarct size and genotype with heterozygotes displaying infarct sizes between those of the wild type and homozygous phenotypes. Secondly, bolus injection of human α₂-AP (hα₂-AP) in α₂-AP^{-/-} mice caused a dose-related infarct expansion. Importantly, Fab fragments from affino-specific polyclonal rabbit anti-hα₂-AP antibodies, given intravenously 40 min after occlusion of the MCA, significantly reduced the cerebral ischemic infarct size. This observation suggests a potential avenue to counteract focal ischemic infarction with the use of α₂-AP inhibitors (e.g. neutralizing monoclonal antibodies or compounds neutralizing α₂-AP activity). This approach was confirmed by infusion of plasmin in mice with MCA occlusion which, by neutralizing α₂ -AP, significantly reduced infarct size. The concentration of α₂-AP in human plasma is 1 mM (12), corresponding to a total body pool of approximately 500 mg. An equivalent dose of a monoclonal Fab fragment would be approximately 400 mg, and that of plasmin approximately 500 mg, which is high but not excessive for single therapeutic administration. Furthermore, the observation that infarct size is proportional to the α₂-AP level (derived from the gene dose effect and the dose-response) suggests that a partial reduction of the plasma level might have a significant beneficial effect.

In view of the above the invention thus relates to the use of compounds that reduce α₂-AP activity in vivo for the treatment of focal cerebral ischemic infarction (ischemic stroke).

In a specific embodiment of the invention use is made of compounds that reduce the circulating α₂-AP concentration. A lower concentration of α₂-AP will lead to a lower activity. In an alternative embodiment, the activity of circulating α₂-AP is reduced directly.

Compounds that are suitable for the reduction of α₂-AP concentration and activity are for example α₂-AP neutralizing antibodies or derivatives thereof. Preferred antibodies are monoclonal antibodies. Derivatives are preferably Fab fragments, scFv fragments.

Compounds neutralizing α₂-AP are for example plasmin, mini-plasmin (lacking the first 4 kringles) or micro-plasmin (lacking all five kringles).

The present invention will be demonstrated in more detail in the following examples, which are however not intended to be limiting to the scope of the invention. In the examples reference is made to the following drawings:
Figures 1 to 3 are histograms comparing the volume (in mm³) of focal cerebral ischemic infarcts after ligation of the middle cerebral artery (MCA) in mice. The data represent mean values and the vertical bars SEM, with the number of experiments given in the columns.
Figure 1 shows the effect of deficiency of plasminogen system components (genotype in abscissa) on focal ischemic cerebral infarct size (in mm³) WT: wild type (pooled values of 50% C57BL6/50% S129, 100% C57BL6 and 100% S129 genetic background).
Figure 2 shows the effect of adenoviral transfer of the t-PA or PAI-1 genes on focal ischemic cerebral infarct size in t-PA or PAI-1 deficient mice, respectively.
Figure 3 shows the effect of α₂-AP on focal ischemic cerebral infarct size.
   A. Effect of α₂-AP genotype on cerebral infarct size.
   B. Effect of injection of hα₂-AP or of hαα₂-AP followed by anti-hα₂-AP Fab fragments on cerebral infarct size.

### EXAMPLES

### EXAMPLE 1

### Murine cerebral ischemic infarction model

### 1. Introduction

All mice included in the present study were generated and bred at the Specific Pathogen Free Facility of the Center for Transgene Technology and Gene Therapy, Campus Gasthuisberg, K.U. Leuven. Gene inactivation was obtained by homologous recombination in embryonic stem cells targeting the genes encoding tissue-type plasminogen activator (t-PA) (13), urokinase-type plasminogen activator (u-PA) (13), plasminogen activator inhibitor-1 (PAI-1) (14, 15), plasminogen (Plg) (16), or α₂-antiplasmin (α₂-AP) (17), as previously described. Mice with inactivated genes encoding u-PA receptor (u-PAR) (18) were not included because of the normal results obtained with u-PA deficient mice.

### 2. Materials and methods

### 2.1 Materials

Human α₂-AP was prepared from fresh frozen plasma as previously described (19).

Polyclonal antisera were raised in rabbits by subcutaneous injection of 200 mg purified human α₂-AP suspended in complete Freund's adjuvant, followed at two biweekly intervals by injection of the antigen suspended in incomplete Freund's adjuvant. Serum was obtained by repeated ear vein puncture. Pooled sera were chromatographed on Protein-A Sepharose (0.5 ml serum per ml wet gel), equilibrated with 0.1 M Tris.HCl, pH 8.1 and IgG eluted with 0.1 M glycine.HCl, pH 2.8, yielding approximately 10 mg protein per ml serum. Affino-specific antibodies were obtained from the dialyzed IgG pool by chromatography on a CNBr-activated Sepharose column substituted with human α₂-AP (2.5 mg/ml wet gel) and eluted with 0.1 M glycine.HCl, pH 2.8, yielding approximately 0.1 mg specific IgG per mg applied.

Fab fragments were obtained from the affino-specific IgG by digestion with papain. Therefore IgG was dissolved to a concentration of 5 mg/ml and digested with 1 percent (w/w) papain in the presence of 50 mM cysteine, 1 mM EDTA, 0.1 M phosphate buffer, pH 7.0 for 5 hours. The reaction was arrested by addition of iodoacetamide to a final concentration of 75 mM. After dialysis the mixture was purified on a protein A Sepharose column equilibrated with PBS. Fab concentration was determined by ELISA calibrated against an IgG standard. SDS gel electrophoresis essentially revealed homogeneous Fab fragments (not shown).

### 2.2 Production of adenoviral vectors

The recombinant adenoviruses AdCMVt-PA and AdCMVPAI-1 were generated by homologous recombination in 293 cells essentially as previously described (20). For AdCMVt-PA, an XbaI-fragment of the plasmid pSTEt-PA encoding wild type human t-PA was ligated into XbaI-digested pACCMVpLpA (21) to produce pACCMVt-PA. The adenovirus precursor pACCMVPAI-1 was generated by ligating the 1.4-kb EcoRI/BglII fragment of pPAI-1RBR containing the entire coding sequence of human PAI-1 into EcoRI/BamHI-digested pACCMVpLpA. In these plasmids, the t-PA and PAI-1 cDNA are positioned between the human cytomegalovirus immediate-early enhancer/promoter and the SV40 t-antigen intron/polyadenylation signal to form a complete transcriptional unit.

Monolayer cultures of 293 cells (22) were cotransfected with 10 mg of pACCMVt-PA or pACCMVPAI-1 and 5 mg of pJM17 (20), a plasmid containing a full-length adenovirus 5 dl309 genome. Homologous recombination between these plasmids results in the formation of recombinant viral genomes in which the adenovirus E1 region is replaced by the respective t-PA or PAI-1 transgenes. Replication of the recombinant viruses in cultured 293 cells is supported by E1A gene products supplied in trans from a copy of E1 integrated into the 293 cell genome.

After transfection, recombinant viral plaques were harvested and amplified as described (23). The identity of recombinant viruses was determined by restriction analysis and Southern blotting of viral DNA prepared from productively infected 293 cells. The recombinant adenovirus AdRR5, which lacks an inserted gene in the E1 position, was generated from pACRR5 and pJM17 in the same manner and was used as a control adenovirus (24, 25). Recombinant viruses were replaqued to ensure clonal identity before further use. Large scale production of recombinant adenovirus was performed as described (23). Purified virus was supplemented with 0.1 mg/ml sterile bovine serum albumin (BSA), snap frozen in liquid nitrogen and stored at -80°C until use. The titer of infectious viral particles in purified stocks was determined by plaque assay on monolayers of 293 cells with 1 hour of adsorption at 37°C. Purified viral stocks of >10¹⁰ plaque forming units (pfu) per ml were routinely obtained. The kinetics and organ distribution of t-PA and PAI-1 expression following adenoviral transfer by intravenous bolus injection have been described elsewhere (26, 27).

### 2.3 Preparation of human plasmin

Human plasminogen was prepared from fresh frozen human blood bank plasma, essentially as described previously (28). Human plasma (6 liter), to which 20 units aprotinin (Trasylol, Bayer, Germany) was added per ml, was cleared by centrifugation at 4,000 rpm for 15 min at 4°C. Lysine-Sepharose (200g wet weight, substitution level approximately 1 mg lysine per g wet Sepharose gel) was added to the supernatant, the mixture stirred for 1 hour at 4°C and the gel recovered on a Buchner funnel. Then 120 g Lysine-Sepharose was added to the filtrate, the mixture stirred and the gel recovered as above. The combined gel fractions were washed with 18 liter 0.2 M K₂HPO₄/KH₂PO₄ buffer, pH 7.5, containing 10 units aprotinin per ml, then poured into a 5 x 60 cm column and washed with 0.02 M NaH₂PO₄, 0.1 M NaCl buffer, pH 7.5, containing 10 units/ml aprotinin at 4°C until the absorbance of the wash fluid at 280 nm was less than 0.05. The column was then eluted with wash buffer containing 0.05 M 6-aminohexanoic acid and protein containing fractions pooled. From 6 liter plasma approximately 145 ml fluid containing 650 mg protein was obtained. The pool was concentrated 2.5-fold on an Amicon PM10 filter and gel filtered on a 5 x 90 cm column of ultragel AcA44 equilibrated with 0.02 M NaH₂PO₄, 0.1 M NaCl buffer, pH 7.5, at a rate of 60 ml per hour. The main peak, containing approximately 590 mg protein was concentrated on an Amicon PM10 filter to a concentration of 10 mg/ml and frozen until use.

Human plasmin was prepared from plasminogen as follows. Lysine-Sepharose (20 g wet gel) was added to human plasminogen (200 mg) solution, the mixture stirred for 3 hours at 4°C, the gel washed on a Buchner funnel and resuspended in 30 ml 0.1 M NaH₂PO₄ buffer, pH 7.4. Urokinase (500 µl of a 50 µM solution, prepared by activation of Saruplase (Grünenthal, Aachen, Germany) with Plasmin.Sepharose was added and the mixture stirred for 15 hours at 4°C. The gel was then washed on a Buchner funnel with 0.1 M NaH₂PO₄ buffer, pH 7.4, poured into a 1.5 x 16 cm column, washed with 0.1 M NaH₂PO₄ buffer, pH 7.4 until the absorbance at 280 nm of the wash fluid was less than 0.05, and eluted with 0.1 M NaH₂PO₄ buffer containing 0.05 M 6-aminohexanoic acid. The protein containing fractions were pooled, glycerol was added to a final concentration of 10 percent and the pool was dialyzed at 4°C against 0.1 M NaH₂PO₄ buffer containing 10 percent glycerol. The final recovery was 25 ml solution with a protein concentration of 4.0 mg/ml and an active plasmin concentration of 25 µM.

### 2.4 Measurement of α₂-antiplasmin in plasma

α₂-Antiplasmin levels in murine plasma were measured by a chromogenic substrate assay, based on its rapid inhibition of plasmin (29). Briefly 10 µl mouse plasma (diluted 1/10 in 0.05 M NaH₂PO₄ buffer, pH 7.4, containing 0.01% Tween 20) is mixed at 37°C with 420 µl 0.05 Tris HCl, 0.1 M NaCl buffer, pH 7.4, containing 0.01% Tween 20, and with 20 µl of 0.125 µM human plasmin (final concentration 5 nM). After 10s incubation, 50 µl of 3 mM S2403 (Chromogenics, Antwerp, Belgium) is added and the change in absorbance measured at 405 nm. The change in absorbance is approximately 0.18 min⁻¹ with buffer and 0.09 min⁻¹ with pooled murine plasma.

### 2.5 Animal experiments

Animal experiments were conducted according to the guiding principles of the American Physiological Society and the International Committee on Thrombosis and Haemostasis (30).

Focal cerebral ischemia was produced by persistent occlusion of the MCA according to Welsh et al. (31). Briefly, mice of either sex, weighing 20 to 30 g, were anesthetized by intraperitoneal injection of ketamine (75 mg/ml, Apharmo, Arnhem, The Netherlands) and xylazine (5 mg/ml, Bayer, Leverkusen, Germany). Atropine (1 mg/kg; Federa, Brussels, Belgium) was administered intramuscularly, and body temperature was maintained by keeping the animals on a heating pad. A "U" shape incision was made between the left ear and left eye. The top and backside segments of the temporal muscle were transsected and the skull was exposed by retraction of the temporal muscle. A small opening (1 to 2 mm diameter) was made in the region over the MCA with a hand-held drill, with saline superfusion to prevent heat injury. The meningae were removed with a forceps and the MCA was occluded by ligation with 10-0 nylon thread (Ethylon, Neuilly, France) and transsected distally to the ligation point. Finally, the temporal muscle and skin were sutured back in place.

AdCMVt-PA, AdCMVPAI-1 or AdRR5 were given as an intravenous bolus of 1.3 x 10⁹ plaque forming units (p.f.u.) 4 days before ligation of the MCA. Human α₂-AP (hα₂-AP) was given intravenously divided in 2 injections, given 1 min before and 30 min after ligation of the MCA, respectively. Fab fragments were injected intravenously as a bolus, 10 min after the second hα₂-AP injection. Human plasmin was given intravenously as a bolus, either 15 min before or 15 min after ligation of the MCA.

The animals were allowed to recover and were then returned to their cages. After 24 hours, the animals were sacrificed with an overdose of Nembutal (500 mg/kg, Abbott Laboratories, North Chicago, IL) and decapitated. The brain was removed and placed in a matrix for sectioning in 1 mm segments. The sections were immersed in 2% 2,3,5-triphenyltetrazolium chloride (TTC) in saline (32), incubated for 30 min at 37°C, and placed in 4 % formalin in phosphate buffered saline. With this procedure, the necrotic infarct area remains unstained (white) and is clearly distinguishable from stained (brick red) viable tissue. The sections were photographed and subjected to planimetry. The infarct volume was defined as the sum of the unstained areas of the sections multiplied with their thickness.

The data are represented as mean ± SEM of n determinations. The significance of differences was determined using analysis of variance followed by Fisher's PLSD test, using the Statview software package or by Student's t-test.

### EXAMPLE 2

### Cerebral ischemic infarct size in mice with targeted inactivation of genes encoding plasminogen system components

Ligation of the left MCA induced a cerebral infarct with a volume of 7.6 ± 1.1 mm³ (n= 11) in wild type mice with a mixed (50%) S129 and (50%) C57BL/6 genetic background, of 9.3 ± 2.7 mm³ (n= 6) in inbred C57BL/6 mice and of 6.4 ± 1.3 mm³ (n= 6) in inbred S129 mice (p= NS versus mixed background, results not shown).

Inactivation of the t-PA gene was associated with a significant reduction of infarct size to 2.6 ± 0.80 mm³ (n= 11), (P< 0.0001 vs wild type mice), whereas inactivation of the u-PA gene had no effect on infarct size (7.8 ± 1.0 mm³, n= 8, p= NS vs wild type).

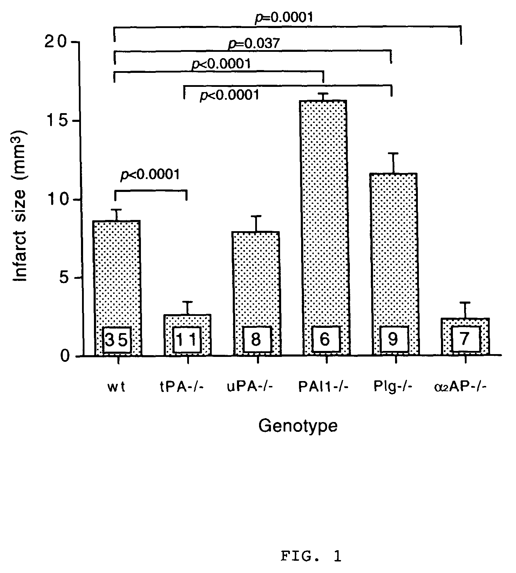

Inactivation of the PAI-1 gene was associated with a significant increase in infarct size (16 ± 0.52 mm³, n= 6, P< 0.0001 vs wild type) (Figure 1). In mice with inactivated Plg genes, cerebral infarct size was significantly larger than in wild type mice (12 ± 1.2 mm³, n=9, p=0.037 vs wild type), whereas, conversely, in α₂-AP gene deficient mice, infarct size was markedly reduced (2.2 ± 1.1 mm³, n= 7, p= 0.0001 vs wild type) (Figure 1).

### EXAMPLE 3

### Effect of t-PA and PAI-1 gene transfer on cerebral infarct size

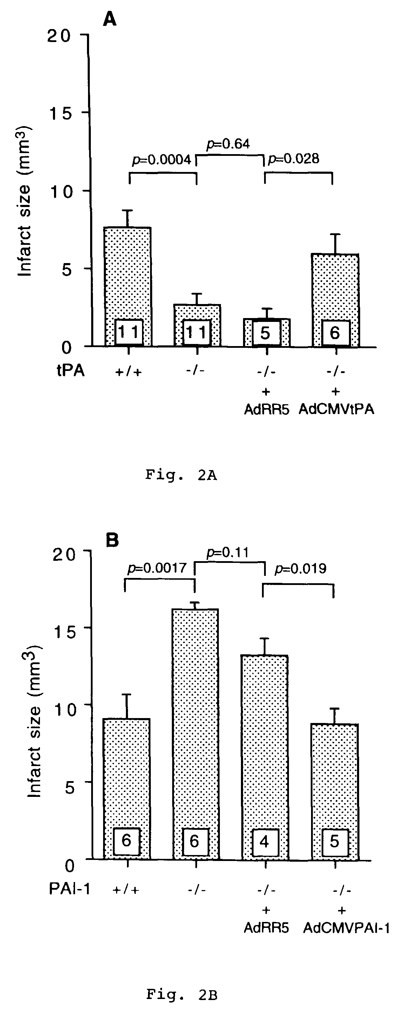

Injection of 1.3 x 10⁹ p.f.u. of AdCMVt-PA in 6 t-PA^{-/-} mice 4 days before MCA ligation was associated with a cerebral infarct size of 6.0 ± 1.3 mm³, significantly larger than the infarcts in 5 t-PA^{-/-} mice injected with the control virus AdRR5 (1.8 ± 0.63, p= 0.028) (Figure 2A). Conversely, injection of 1.3 x 10⁹ p.f.u. of AdCMVP-AI-1 in 5 PAI-1^{-/-} mice was associated with a cerebral infarct size of 10 ± 1.4 mm³, significantly smaller than the infarcts in 5 PAI-1^{-/-} mice injected with the control virus AdRR5 (13 ± 1.0 mm³, p= 0.019) (Figure 2B).

### EXAMPLE 4

### Effect of α₂-antiplasmin on cerebral infarct size

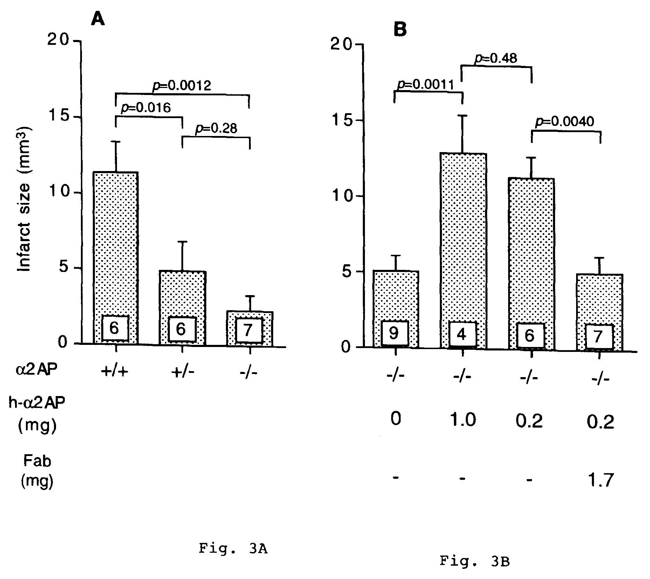

Cerebral infarct size correlated with α₂-AP gene dose, corresponding to 11 ± 2.0, 4.9 ± 2.0 and 2.2 ± 1.1 mm³ in wild type, heterozygous and homozygous deficient mice, respectively (Figure 3A). Injection of human α₂-AP in groups of 4 α₂-AP^{-/-} mice increased the infarct size to 13 ± 2.5 mm³ (n= 4) with a 1 mg total dose and to 11 ± 1.5 mm³ (n= 6) with a 0.2 mg total dose. Injection of 1.7 mg affino-specific Fab against human α₂-AP in mice given 0.2 mg human α₂-AP reduced the cerebral infarct size to 5.1 ± 1.1 mm³ (n= 7, p= 0.0040 vs 0.2 mg human α₂-AP) (Figure 3B).

The above examples show that reduction of α₂-AP activity (reduced α₂-AP gene expression or reduction of circulating α₂-AP with inhibitors) reduces focal cerebral ischemic infarct size, such as encountered during ischemic stroke.

### EXAMPLE 5

### Effect of plasmin on cerebral infarct size

Injection of 50, 100 or 150 µg human plasmin (Pli) in mice weighing approximately 30 g decreased the α₂-AP levels in blood samples taken after 30 s to 67, 40 and 31 percent of baseline, respectively (mean of 2 mice, with less than 15 percent variability). Injection of 200 µg Pli in 3 mice reduced the plasma α₂-AP levels to 59 ± 4.8, 67 ± 4.4 and 70 ± 2.5 percent after 2, 4 and 6 hours respectively.

Ligation of the left middle cerebral artery (MCA) induced a cerebral infarct with a volume of 27 ± 1.3 mm³ (n= 10) in inbred Balb/c mice, and of 16 ± 1.3 mm³ (n= 12) in inbred C57BL/6 mice.

Injection of 0.2 mg Pli in Balb/c mice reduced the infarct size to 22 ± 1.0 mm³ (n= 9) (p= 0.006 vs saline). Similar decreases were observed when the Pli injection was given 15 min before or 15 min after ligation of the MCA (Table 1) . In C57Bl/6 mice, injection of 0.2 mg Pli reduced the infarct size to 10 ± 1.2 mm³ (n= 12) (p= 0.004 vs saline).

### REFERENCES

1. Collen D. Staphylokinase: a potent, uniquely fibrin-selective thrombolytic agent. Nature Medicine 1998; 4: 279-284.
2. Coyle JT, Puttfarcken P. Oxidative stress, glutamate and neurodegenerative disorders. Science 1993; 262: 689-695.
3. Coyle JT, Molliver ME, Kuhar MJ. In situ injection of kainic acid: a new method for selectively lesioning neuronal cell bodies while sparing axons of passage. J Comp Neurol 1978; 180: 301-323.
4. Tsirka S, Gualandris A, Amaral DG, Strickland S. Excitotoxin induced neuronal degeneration and seizure are mediated by tissue plasminogen activator. Nature 1995; 377: 340-344.
5. Tsirka S, Rogove AD, Bugge TH, Degen JL, Strickland S. An extracellular proteolytic cascade promotes neuronal degeneration in the mouse hippocampus. J Neurosci 1997; 17: 543-552.
6. Tsirka S, Rogove AD, Strickland S. Neuronal cell death and t-PA. Nature 1996; 384: 123-124.
7. Chen ZL, Strickland S. Neuronal death in the hippocampus is promoted by plasmin-catalyzed degradation of laminin. Cell 1997; 91: 917-925.
8. Wang YF, Tsirka SE, Strickland S, Stieg PE, Soriano SG, Lipton SA. Tissue plasminogen activator (tPA) increases neuronal damage after focal cerebral ischemia in wild-type and tPA-deficient mice. Nature Medicine 1998; 4: 228-231.
9. Nagai N, Vanlinthout I, Collen D. Comparative effects of tissue-type plasminogen activator, streptokinase and staphylokinase on cerebral ischemic infarction and pulmonary clot lysis in hamster models. Submitted.
10. The National Institute of Neurological Disorders and Stroke rt-PA Stroke Study Group. Tissue plasminogen activator for acute ischemic stroke. N Engl J Med 1995; 333: 1581-1587.
11. Hacke W, Kaste M, Fieschi C, Toni D, Lesaffre E, von Kummer R, Boysen G, Bluhmki E, Haxter G, Mahagne MH, Hennerici M, for the ECASS Study Group. Intravenous thrombolysis with recombinant tissue plasminogen activator for acute hemispheric stroke: the European Cooperative Actue Stroke Study (ECASS). J Am Med Ass 1995; 274: 1017-1025.
12. Collen D, Wiman B. Turnover of antiplasmin, the fast-acting plasmin inhibitor of plasma. Blood 1979; 53: 313-324.
13. Carmeliet P, Schoonjans L, Kieckens L, Ream B, Degen J, Bronson R, De Vos R, van den Oord J, Collen D, Mulligan R. Physiological consequences of loss of plasminogen activator gene function in mice. Nature 1994; 368: 419-424.
14. Carmeliet P, Kieckens L, Schoonjans L, Ream B, Van Nuffelen A, Prendergast G, Cole M, Bronson R, Collen D, Mulligan RC. Plasminogen activator inhibitor-1 gene-deficient mice. I. Generation by homologous recombination and characterization. J Clin Invest 1993; 92: 2746-2755.
15. Carmeliet P, Stassen JM, Schoonjans L, Ream B, Van den Oord JJ, De Mol M, Mulligan RC, Collen D. Plasminogen activator inhibitor-1 deficient mice. II. Effects on hemostasis, thrombosis and thrombolysis. J Clin Invest 1993; 92: 2756-2760.
16. Poplis VA, Carmeliet P, Vazirzadeh S, Van Vlaenderen I, Moons L, Plow EF, Collen D. Effects of disruption of the plasminogen gene on thrombosis, growth and health in mice. Circulation 1995; 92:2585-2593.
17. Lijnen HR, Okada K, Matsuo O, Collen D, Dewerchin M. α₂-antiplasmin gene-deficiency in mice is associated with enhanced fibrinolytic potential without overt bleeding. Blood 1999; 93:2274-2281.
18. Dewerchin M, Van Nuffelen A, Wallays G, Bouché A, Moons L, Carmeliet P, Mulligan RC, Collen D. Generation and characterization of urokinase receptor-deficient mice. J Clin Invest 1996; 97: 870-878.
19. Lijnen HR, Holmes WE, Van Hoef B, Wiman B, Rodriguez H, Collen D. Amino-acid sequence of human α₂-antiplasmin. Eur J Biochem 1987; 166: 565-574.
20. McGrory WJ, Bautista DS, Graham FL. A simple technique for the rescue of early region 1 mutations into infectious human adenovirus type 5. Virology 1988; 163: 614-617.
21. Gomez-Foix AM, Coats WS, Baque S, Alam T, Gerard RD, Newgard CB. Adenovirus-mediated transfer of the muscle glycogen phosphorylase gene into hepatocytes confers altered regulation of glycogen metabolism. J Biol Chem 1992; 267: 25129-25134.
22. Graham FL, Smiley J, Russel WC, Nairn R. Characteristics of a human cell line transformed by DNA from human adenovirus type 5. J Gen Virol 1977; 36: 59-74.
23. Gerard RD, Meidell RS. Adenovirus vectors. In: Hames BD, Glover D (eds): DNA Cloning - A practical approach: mammalian systems. Oxford, UK, 1995, p 285-307.
24. Alcorn JL, Gao E, Chen Q, Smith ME, Gerard RD, Mendelson CR. Genomic elements involved in transcriptional regulation of the rabbit surfactant protein-A gene. Mol Endocrinol 1993; 7: 1072-1085.
25. Kopfler WP, Willard M, Betz T, Willard JE, Gerard RD, Meidell RS. Adenovirus-mediated transfer of a gene encoding human apolipoprotein A-Iinto normal mice increases circulating high-density lipoprotein cholesterol. Circulation 1994; 90: 1319-1327.
26. Carmeliet P, Stassen JM, Van Vlaenderen I, Meidell RS, Collen D, Gerard RD. Adenovirus-mediated transfer of tissue-type plasminogen activator augments thrombolysis in tissue-type plasminogen activator-deficient and plasminogen activator inhibitor-1-overexpressing mice. Blood 1997; 90: 1527-1534.
27. Carmeliet P, Moons L, Lijnen R, Janssens S, Lupu F, Collen D, Gerard RD. Inhibitory role of plasminogen activator inhibitor-1 in arterial wound healing and neointima formation. Circulation 1997; 96: 3180-3191.
28. Deutsch DG, Mertz ET. Plasminogen purification from human plasma by affinity chromatography. Science 1970; 170: 1095-1096.
29. Edy J, De Cock F, Collen D. Inhibition of plasmin by normal and antiplasmin-depleted human plasma. Thromb Res. 1976; 8: 513-518.
30. Giles AR. Guidelines for the use of animals in biomedical research. Thromb Haemost 1987; 58: 1078-1084.
31. Welsh FA, Sakamoto T, McKee AE, Sims RE. Effect of lactacidosis on pyridine nucleotide stability during ischemia in mouse brain. J Neurochem 1987; 49: 846-851.
32. Bederson JB et al. Evaluation of 2,3,5-triphenyltetrazolium chloride as a stain for detection and quantification of experimental cerebral infarction in rats. Stroke 1986; 17: 472-476.

* Present address: E-317, Handa-cho 3776, Hamamatsu, Shizuoka 431-3124, Japan
17

## Claims

1. Use of compounds that reduce α₂-antiplasmin in vivo, for the preparation of a therapeutical composition for the treatment of focal cerebral ischemic infarction (ischemic stroke).

2. The use according to claim 1, wherein the compounds reduce the circulating α₂-antiplasmin concentration.

3. The use according to claim 1, the compounds reduce the circulating α₂-antiplasmin activity.

4. The use according to claims 1-3, wherein the compounds are α₂-antiplasmin neutralizing antibodies or derivatives thereof.

5. The use according to claim 4, wherein the derivatives are Fab fragments or ScFv fragments.

6. The use according to claims 1-3, wherein the compounds are α₂-antiplasmin neutralizing compounds selected from plasmin, mini-plasmin (lacking the first four kringles) or micro-plasmin (lacking all five kringles).
